# EUROPEAN PATENT APPLICATION

(11) **EP 3 560 514 A1**
(43) Date of publication of application: **30.10.2019**
(21) Application number: 17887644.7
(22) Date of filing: 26.12.2017
(51) Int. Cl.: A61K 45/00, A61K 31/16, A61K 31/404, A61K 31/519, A61K 31/683, A61K 31/685, A61K 31/717, A61K 31/718, A61K 31/722, A61K 31/734, A61K 36/73, A61K 36/899, A61K 38/00, A61K 39/395, A61P 5/00, A61P 43/00, C07K 16/26

(54) **HYPOTHERMIA AMELIORATING AGENT**

(30) Priority: 26.12.2016 JP 2016251776
(71) Applicant: Kao Corporation, Chuo-Ku Tokyo 103-8210 (JP)
(72) Inventor: OKAHARA, Fumiaki, Haga-gun Tochigi 321-3497 (JP); MORI, Takuya, Haga-gun Tochigi 321-3497 (JP)
(74) Representative: Vossius & Partner Patentanwälte Rechtsanwälte mbB
(86) International application number: PCT/JP2017/046524
(87) International publication number: WO 2018/124009

(57) **Abstract**

Provided is a hypothermia preventing or ameliorating agent useful for hypothermia. The hypothermia preventing or ameliorating agent comprises a GIP function inhibitor as an active ingredient.

## Description

### Field of the Invention

The present invention relates to a hypothermia preventing or ameliorating agent.

### Background of the Invention

The average human body temperature is approximately 36.5°C, which is the temperature at which enzymes in the body are most activated to function.

However, in recent years, the number of humans with hypothermia whose normal temperature is dropped by 0.5°C to 1.5°C is increasing. The causes thereof are thought to be, for example, a shortage of minerals and vitamins or protein, a lack of exercise, or poor blood circulation or a disturbance of the autonomic nervous system due to excessive stress. In addition, the human normal temperature is the highest in the babyhood stage and gradually decreased with advancing age. Although the decrease in the body temperature stops in the adolescence, the body temperature starts to be decreased again in the elderly and tends to be about 0.2°C lower than that when young. As one of factors thereof, hypofunction of brown adipose tissue responsible for thermogenesis is considered.

Such hypothermia leads to a decrease in immunity, a decrease in basal metabolism, and a decrease in enzymatic activity in the body and tends to increase the susceptibility to infectious diseases such as colds and allergic symptoms such as hay fever. In addition, poor blood circulation, a decrease in the flow of lymph or metabolism, or imbalance of the autonomic nervous system is caused. Accordingly, in hypothermia, symptoms, such as various symptoms due to functional disorders of internal organs, diarrhea and dehydration symptoms, cold hands and feet, stiff shoulders, headaches, back pain, abdominal pain, menstrual pain, agrypnia, a decrease in the quality of sleep, tired feeling, and a decrease in activity, often appear.

GIP (gastric inhibitory polypeptide or glucose-dependent insulinotropic polypeptide) is one of gastrointestinal hormones belonging to the glucagon/secretin family. GIP is called incretin, as with GLP-1 (glucagon-like peptide 1), and is secreted by K cells present in the small intestine upon intake of lipids or carbohydrates.

It is known that GIP promotes insulin secretion from pancreatic β cells and enhances uptake of glucose into fat cells in the presence of insulin. Accordingly, the action of GIP is considered to be partly responsible for obesity. It has been reported that obesity is actually suppressed by inhibiting the function of GIP (Non Patent Literature 1).

Furthermore, it has been reported that GIP is partly responsible for insulin resistance (Non Patent Literature 1). When insulin resistance occurs, glucose-absorbing effect mediated by insulin is reduced, as a result, causing hyperinsulinemia. Hyperinsulinemia is recognized to be a primary cause leading to occurrence of various lifestyle-related diseases including obesity, and prevention and amelioration of insulin resistance are important also from the aspect of reducing the risk of lifestyle-related diseases.

However, there is no report that GIP has a relation with hypothermia, and it is not known at all that hypothermia can be ameliorated by decreasing the blood GIP concentration.

(Non Patent Literature 1) Miyawaki K., et al., Nat. Med. 8(7): 738-42, 2002

### Summary of the Invention

The present invention relates to the following aspects 1) to 5):
1) a hypothermia preventing or ameliorating agent comprising a GIP function inhibitor as an active ingredient;
2) use of a GIP function inhibitor for producing a hypothermia preventing or ameliorating agent;
3) a GIP function inhibitor for use in prevention or amelioration of hypothermia;
4) use of a GIP function inhibitor for preventing or ameliorating a hypothermia; and
5) a method for preventing or ameliorating hypothermia, comprising administering a GIP function inhibitor to a subject in need thereof.

### Brief Description of Drawings

Figure 1 is a calibration curve for a sandwich ELISA using an anti-active GIP antibody.
Figure 2 is a graph showing acute changes in the deep body temperature after administration of GIP.
Figure 3 is a graph showing chronic changes in the deep body temperature by continuous administration of GIP or a GIP-binding anti-GIP antibody.
Figure 4 is a graph showing age-related changes in the blood GIP level.
Figure 5 is a graph showing chronological changes in the deep body temperature with aging.
Figure 6 is a graph showing chronic changes in the deep body temperature by continuous administration of an anti-GIP antibody to aged mice.
Figure 7 is a graph showing the deep body temperatures after continuous intake of a GIP receptor antagonist and a GIP secretion suppressing agent.

### Detailed Description of the Invention

The present invention relates to provide a hypothermia ameliorating agent useful for ameliorating hypothermia.

The present inventors examined the relationship between GIP and body temperature and found that body temperature is significantly decreased by administration of GIP and hypothermia can be ameliorated by suppressing the GIP function.

The hypothermia preventing or ameliorating agent of the present invention shows an effect of suppressing a decrease in body temperature in hypothermia conditions, for example, hypothermia with aging. Accordingly, it is useful for preventing or ameliorating a decrease in immunity, a decrease in basal metabolism, poor blood circulation, a decrease in the flow of lymph or metabolism1, imbalance of the autonomic nervous system, or the like that are caused by hypothermia.

In the present invention, GIP (gastric inhibitory polypeptide or glucose-dependent insulinotropic polypeptide) is a polypeptide consisting of 42 amino acids represented by SEQ ID NO: 1. GIP(1-42) has physiological activity (active GIP), but becomes inactive GIP (3-42) by cleavage of two amino acids at the N-terminus with dipeptidyl peptidase-4 (DPP-4) present in vivo.

In the present invention, the "GIP function inhibitor" means a substance that inhibits or suppresses the function of GIP as a gastrointestinal hormone, i.e., a substance that inhibits the function at the GIP gene or GIP receptor gene level or at the GIP itself or GIP receptor level. Specifically, the inhibitor is, for example, an anti-GIP antibody, a GIP receptor antagonist, or a GIP secretion or increase-suppressing agent.

In the present invention, the "anti-GIP antibody" may be any antibody that at least inhibits the function of active GIP and may be a polyclonal antibody or a monoclonal antibody and preferably an antibody that substantially does not bind to inactive GIP (referred to as "anti-active GIP antibody") described in WO 2016/104439 and JP-A-2013-138638. The binding constant (Ka) with active GIP is preferably 10⁷ M⁻¹ or more, more preferably 10⁸ M⁻¹ or more, even more preferably 10⁹ M⁻¹ or more.

The anti-active GIP antibody incudes antibodies in which the amount of a test antibody bound to inactive GIP is 10% or less at most, preferably 5% or less, more preferably 1% or less, even more preferably 0.1% when the amount of the test antibody bound to active GIP is assumed to be 100%. The amount of the test antibody bound to inactive GIP can be determined by measuring the binding between the test antibody and inactive GIP through a method such as western blotting, immunoprecipitation, immunohistochemical staining, or ELISA.

The anti-active GIP antibody is, for example, an antibody recognizing the 8th and subsequent amino acids from the N-terminus of active GIP (SEQ ID NO: 5) and is preferably an antibody recognizing one or more amino acids selected from at least the 8th to 10th amino acids (SDY).

The anti-active GIP antibody is preferably an antibody further including a region consisting of the amino acid sequence represented by the following formula (1) or a conservative sequence modification thereof in an H-chain.

### EMNPSDGRTHFNE (1)

The alphabetical letters in formula (1) mean the one-letter codes of amino acids, and the sequence is shown in order from the N-terminus to the C-terminus. Here, F is phenylalanine, T is threonine, D is aspartic acid, E is glutamic acid, M is methionine, N is asparagine, P is proline, S is serine, G is glycine, R is arginine, and H is histidine.

In the present specification, the "conservative sequence modification" is an amino acid modification in a region other than the complementarity determining region (CDR) participating in antigen determination, and means amino acid modification that does not significantly affect or change the reactivity of the antibody consisting of the unmodified amino acid sequence. Such conservative sequence modification encompasses substitution, addition, and deletion of one to several, preferably 1 to 3, more preferably one amino acid. The conservatively modified amino acid sequence has, for example, a sequence identity of 90% or more, preferably 95% or more, more preferably 99% or more with the unmodified amino acid sequence. The modification can be introduced into the antibody of the present invention by a standard technique known in the art, such as site-directed mutagenesis or PCR-mediated mutagenesis. Examples of the conservative amino acid substitution include substitution of an amino acid residue with an amino acid residue having a similar side chain (a family of the amino acid residue). Such families of amino acid residues are defined in the art and include amino acids having basic side chains (e.g., lysine, arginine, and histidine), acid side chains (e.g., aspartic acid and glutamic acid), uncharged polar side chains (e.g., glycine, asparagine, glutamine, serine, threonine, tyrosine, cysteine, and tryptophan), nonpolar side chains (e.g., alanine, valine, leucine, isoleucine, proline, phenylalanine, and methionine), β-branched side chains (e.g., threonine, valine, and isoleucine), and aromatic side chains (e.g., tyrosine, phenylalanine, tryptophan, and histidine).

The identity between amino acid sequences refers to the ratio (%) of the number of positions at which the identical amino acid residues are present in both sequences relative to the number of full-length amino acid residues when the two amino acid sequences are aligned. Specifically, for example, the identity can be calculated by the Lipman-Pearson method (Science, 227, 1435, (1985)) and determined by analysis using a homology analysis (Search homology) program of genetic information processing software Genetyx-Win (Ver.5.1.1; Software Development) with setting Unit size to compare (ktup) at 2.

The amino acid sequence represented by formula (1) described above encodes the region consisting of 13 amino acid residues at the 50th to 62nd positions of the amino acid sequence represented by SEQ ID NO: 2 representing the H-chain variable region.

Accordingly, the anti-active GIP antibody more preferably includes a region consisting of the amino acid sequence represented by SEQ ID NO: 2 or a conservative sequence modification thereof as the H-chain variable region. Furthermore, the anti-active GIP antibody more preferably includes a region consisting of the amino acid sequence represented by SEQ ID NO: 2 or a conservative sequence modification thereof as the H-chain variable region and a region consisting of the amino acid sequence represented by SEQ ID: 4 or a conservative sequence modification thereof as the L-chain variable region.

Examples of the anti-active GIP antibody including a region consisting of the amino acid sequence represented by SEQ ID NO: 2 as the H-chain variable region and a region consisting of the amino acid sequence represented by SEQ ID NO: 4 as the L-chain variable region include the monoclonal antibody produced by hybridoma 9B9H5-B9 line shown in Production Example 1 described later.

The anti-GIP antibody of the present invention may be a fragment of the antibody, , such as F(ab')₂, F(ab'), single chain Fv (scFv), disulfide-linked Fv (dsFv) in which amino acid residues substituted for the cysteine residues in the VH and the VL are linked to each other through a disulfide bond, or a polymer thereof, or a dimerized V region (Diabody) in which scFv is dimerized as long as the fragment has the reactivity. Furthermore, the fragment of the antibody may be a peptide including a part of the anti-active GIP antibody, as long as the peptide has the reactivity, and specifically includes a peptide including a part of the amino acid sequence constituting the antibody and having the reactivity.

In addition, the immunoglobulin class of the anti-GIP antibody of the present invention is not particularly limited and may be any of IgG, IgM, IgA, IgE, IgD, and IgY immunoglobulin classes and is preferably IgG. The antibody of the present invention encompasses antibodies of any isotype.

In addition, the anti-GIP antibody (including an anti-active GIP antibody) of the present invention may be any one of antibodies of non-human animals, human chimeric antibodies, humanized antibodies, and human antibodies. Examples of the antibodies of non-human animals include antibodies of mouse, rat, hamster, and guinea pig, and mouse antibodies are preferred.

Here, the "human chimeric antibody" is an antibody modified by genetic engineering such that the constant region of an antibody derived from a non-human animal and specifically binding to GIP is replaced with the corresponding constant region of a human antibody, and is preferably a human-mouse chimeric antibody. The "humanized antibody" is an antibody modified by genetic engineering such that the primary structure except for the H chain and L chain complementarity determining region (CDR) of an antibody derived from a non-human animal and specifically binding to GIP is replaced with the corresponding primary structure of a human antibody. The "human antibody" means a human antibody that is an expression product of a completely human-derived antibody gene.

The anti-GIP antibody that can be used is a monoclonal antibody produced by a known method, in addition to a commercially available polyclonal antibody (Bioss Inc.). Examples of the monoclonal antibody derived from a mammal include those produced by hybridomas and those produced by a well-known genetic engineering technique using a designed antibody gene or antibody fragment gene.

For example, the anti-active GIP antibody described above is produced as a recombinant single-chain antibody protein (scFv) having antigen binding ability by inserting a DNA encoding an H-chain variable region (e.g., a DNA consisting of the nucleotide sequence represented by SEQ ID NO: 1) and a DNA encoding an L-chain variable region (e.g., a DNA consisting of the nucleotide sequence represented by SEQ ID NO: 3) into the downstream of a promoter in respective appropriate vectors to construct recombinant vectors, introducing the recombinant vectors into host cells to produce an H-chain and an L-chain from the resultant transformants, and linking the chains via a possible peptide; or by linking a DNA encoding an H-chain variable region (e.g., a DNA consisting of the nucleotide sequence represented by SEQ ID NO: 1) and a DNA encoding an L-chain variable region (e.g., a DNA consisting of the nucleotide sequence represented by SEQ ID NO: 3) via a DNA encoding a known linker, inserting the resultant DNA construct into the downstream of a promoter in an appropriate vector to construct a recombinant vector and expressing the DNA sequence in a host cell (see, for example, MacCfferty, J., et al., Nature, 348, 552-554, 1990; and Tim Clackson, et al., Nature, 352, 642-628, 1991). Furthermore, the anti-active GIP antibody may be produced by linking a DNA encoding a variable region and a DNA encoding a constant region and expressing the DNA sequence. In this case, the constant region and the variable region may be derived from the same antibody or may be derived from a different antibody.

As described above, an amino acid mutant for preparing functionally equivalent polypeptides can be introduced by, for example, site-directed mutagenesis.

An anti-active GIP antibody-producing hybridoma can be basically produced by a known technique as follows.

For example, active GIP or a peptide including an N-terminal amino acid sequence (a peptide consisting of the 1st to 15th amino acids of SEQ ID NO: 5) is linked to an appropriate carrier protein, for example, keyhole limpet hemocyanin (KLH) or bovine serum albumin, as needed, to enhance the immunogenicity and is used for immunization of a non-human mammal to produce the hybridoma. The active GIP or the peptide used as the sensitizing antigen (immunogen) can be produced by genetic engineering or chemical synthesis.

The mammal to be immunized with the sensitizing antigen is not particularly limiter, is preferably selected considering the compatibility with myeloma cells of a mammal as a parent cell to be used for cell fusion and is usually a rodent such as a mouse, a rat, or a hamster.

An animal is immunized with the sensitizing antigen according to a known method. For example, the sensitizing antigen is injected intraperitoneally or subcutaneously into a mammal for immunization. Specifically, the sensitizing antigen is diluted or suspended in, for example, PBS (phosphate-buffered saline) or physiological saline to obtain an appropriate amount, the dilution or suspension is, if desired, mixed with an appropriate amount of a common adjuvant, for example, Freund's complete adjuvant for emulsification. The emulsion is then administered subcutaneously, intradermally, or intraperitoneally to an animal for temporal stimulation, and the same procedure is repeated as needed. The amount of the antigen administered is appropriately determined according to the administration route and the animal species and, usually, is preferably about from 10 µg to 1 mg per once. After confirmation of an increase in the level of the desired antibody in the serum of the animal thus immunized, the immunocytes are taken from the mammal having an increased antibody level and are used for cell fusion. In particular, examples of the immunocyte preferred for the cell fusion include a spleen cell.

As myeloma cells of the mammal serving as the other parent cell to be fused with the immunocytes, various known cell lines, such as P3X63, NS-1, MPC-11, and SP2/0, are appropriately used.

The immunocytes and the myeloma cells can be fused according to a known method, for example, a Kohler's method (Kohler, et al., Nature, vol. 256, p495-497 (1975)). That is, the immunocytes and the myeloma cells are mixed in the presence of a cell fusion promoter, such as polyethylene glycol (PEG having an average molecular weight of 1,000 to 6,000, concentration: 30% to 60%) or hemagglutinating virus of Japan (HVJ), in a nutrient medium, such as a RPMI1640 medium or a MEM medium, containing an auxiliay, such as dimethyl sulfoxide, if desired, to form fused cells (hybridomas).

The hybridomas formed by fusion are cultured in a selection medium, such as a medium containing hypoxanthine, thymidine, and aminopterin (HAT medium), for 1 to 7 days and thereby separated from unfused cells. The resulting hybridomas are subjected to further selection based on a produced antibody (antibody binding to active GIP and not substantially binding to inactive GIP) .

The selected hybridomas are cloned according to a known limiting dilution method to establish a monoclonal antibody-producing hybridoma.

A method for detecting the activity of the antibody produced by the hybridoma can be a known method, such as an ELISA, agglutination, or radioimmunoassay.

In order to obtain a monoclonal antibody from the resulting hybridoma, for example the following methods are adopted: a method which involves culturing the hybridoma according to an ordinary method to obtain the monoclonal antibody as a culture supernatant, or a method which involves administering the hybridoma to a mammal compatible therewith proliferating the hybridoma, and obtaining the monoclonal antibody as an ascitic fluid thereof.

The antibody can be purified by a known purification method, such as a salting-out method, a gel filtration method, ion exchange chromatography, or affinity chromatography.

In the present invention, examples of the "GIP receptor antagonist" include methylidene hydrazide compounds described in WO 2003/097031, specifically, 4-hydroxybenzoic acid (2-bromobenzylidene) hydrazide, 3-cyano-4-hydroxybenzoic acid [1-(2,3,5,6-tetramethylbenzyl)indol-4-yl]methylidene hydrazide, 3-chloro-4-hydroxybenzoic acid (4-methoxynaphthalen-1-yl)methylidene hydrazide, and 3-chloro-4-hydroxybenzoic acid [1-(5-chlorothiophen-2-ylmethyl)-1H-indol-5-yl]methylidene hydrazide.

In the present invention, examples of the "GIP secretion or increase-suppressing agent" include BMPP (3-bromo-5-methyl-2-phenylpyrazolo[1,5-a]pyrimidin-7-ol) (WO 2001/87341), alginic acid (JP-A-2013-166741), phosphatidylethanolamine (JP-A-2010-222284), polyglutamic acid (JP-A-2012-144486), quillaja (JP-A-2012-171914), lysophosphatidylinositol (JP-A-2012-171915), cellulose nanofiber (JP-A-2009-126837), β-chitin nanofiber (JP-A-2010-241713), diacylglycerol (JP-A-2006-342084), hydroxypropylated starch (JP-A-2006-342085), monoacylglycerol (JP-A-2007-290989), a very long chain fatty acid having 20 or more carbon atoms (for example, arachidic acid, behenic acid, lignoceric acid, cerotic acid, montanic acid, melissic acid, lacceric acid, gadoleic acid, dihomo-γ-linolenic acid, arachidonic acid, eicosapentaenoic acid, erucic acid, docosapentaenoic acid, docosahexaenoic acid, nervonic acid, hexacosenoic acid, and octacosenoic acid: JP-A-2011-225458), triacylglycerol containing 1 mass% or more of docosahexaenoic acid and 1 mass% or more of eicosapentaenoic acid as constituent fatty acids (JP-A-2013-063937), long chain unsaturated fatty acid ethanolamide (for example, oleylethanolamide, linoleylethanolamide, linolenylethanolamide, homo-γ-linolenylethanolamide, arachidonylethanolamide, and 7,10,13,16-docosatetraenylethanolamide: JP-A-2010-180203), a rice bran extract (JP-A-2012-515139), a catechin (JP-A-2010-260856), triacylglycerol containing 10 mass% or more α-linolenic acid as a constituent fatty acid (JP-A-2013-075887), and acylglycerol with a C14 to C18 saturated fatty acid bound at the 2-position of the glycerol skeleton (for example, 2-acylmonoglycerol with lauric acid (12:0), myristic acid (14:0), palmitic acid (16:0), linoleic acid (18:2), oleic acid (18:1), stearic acid (18:0), or arachidonic acid (20:4) bound at the 2-position: JP-A-2016-047805).

As shown in Examples described later, the anti-GIP antibody suppresses a decrease in the body temperature caused by administration of GIP and thus has an activity of ameliorating the chronic hypothermia of aged mice.

Accordingly, a GIP function inhibitor such as the anti-GIP antibody, can be a hypothermia preventing or ameliorating agent and can be used for producing a hypothermia preventing or ameliorating agent.

In addition, the GIP function inhibitor can be used for preventing or ameliorating hypothermia. Here, the use can be use for a human being or a non-human animal or in a sample derived therefrom, and may be therapeutic use or non-therapeutic use. The term "non-therapeutic" is a concept that does not include medical practice, i.e., a concept not including a method for operation, treatment, or diagnosis for a human being, more specifically, a concept not including a method for performing operation, treatment, or diagnosis for a human being by a doctor or a person instructed by a doctor.

In the present invention, the term "hypothermia" means that the deep body temperature is decreased by 0.5°C or more than the normal temperature, and the term "prevention or amelioration of hypothermia" means that hypothermia caused by a decrease in the deep body temperature is suppressed or that hypothermia is ameliorated by increasing the deep body temperature. Here, the deep body temperature means the temperature of the deep part of the body (for example, the rectum, esophagus, heart, or brain) and is usually the rectal temperature. The deep body temperature in a human being can be calculated from, for example, the armpit temperature, oral (buccal) temperature, or skin temperature.

Although the cause of hypothermia is, for example, a shortage of minerals and vitamins or protein, a lack of exercise, poor circulation or a disturbance of the autonomic nervous system due to excessive stress, or aging and is not particularly limited, the present invention is suitable for prevention or amelioration of hypothermia caused by aging.

The hypothermia preventing or ameliorating agent of the present invention can be human or veterinary medicine showing an effect of suppressing a decrease in the body temperature or ameliorating hypothermia or a material or preparation to be used by being blended in medicine.

When the hypothermia preventing or ameliorating agent of the present invention is used as medicine, the medicine can be administered in an arbitrary dosage form. Examples of the dosage form include oral administration in the form of, for example, tablets, capsules, granules, powders, and syrups, and parenteral administration in the form of, for example, injections, suppositories, inhalants, transdermal absorbents, and external preparations. Preferred form is parenteral administration.

The medicinal preparations of such various dosage forms can be prepared from the GIP function inhibitor of the present invention alone or in appropriate combination with other pharmaceutically acceptable ingredients, such as an excipient, a binder, a filler, a disintegrant, a surfactant, a lubricant, a dispersant, a buffering agent, a preservative, a corrective agent, a flavor, a coating agent, a carrier, and a diluent.

The content of the GIP function inhibitor in the hypothermia preventing or ameliorating agent of the present invention is preferably 0.001 mass% or more, more preferably 0.01 mass% or more; preferably 1 mass% or less, more preferably 0.1 mass% or less; and is preferably from 0.001 to 1 mass%, more preferably from 0.01 to 0.1 mass%.

The amount of the hypothermia preventing or ameliorating agent of the present invention administered can vary depending on the condition, weight, sex, age, or other factors of the subject. In the case of oral administration or intake, the amount as the GIP function inhibitor is preferably 1 mg or more, more preferably 5 mg or more, and preferably 100 mg or less, more preferably 20 mg or less per day for an adult.

The subject to be administered with the hypothermia preventing or ameliorating agent of the present invention is preferably a human being whose deep body temperature is 36°C or less.

Regarding the above-described embodiments, in the present invention, the following aspects are further disclosed.
<1> A hypothermia preventing or ameliorating agent comprising a GIP function inhibitor as an active ingredient.
<2> Use of a GIP function inhibitor for producing a hypothermia preventing or ameliorating agent.
<3> A GIP function inhibitor for use in prevention or amelioration of hypothermia.
<4> (Non-therapeutic) Use of a GIP function inhibitor for preventing or ameliorating hypothermia.
<5> A method for preventing or ameliorating hypothermia, comprising administering a GIP function inhibitor to a subject in need thereof.
<6> In aspects <1> to <5>, the GIP function inhibitor is an anti-GIP antibody, a GIP receptor antagonist, or a GIP secretion or increase-suppressing agent.
<7> In aspect <6>, the anti-GIP antibody is preferably an anti-active GIP antibody.
<8> In aspect <6>, the GIP receptor antagonist is preferably 4-hydroxybenzoic acid (2-bromobenzylidene) hydrazide, 3-cyano-4-hydroxybenzoic acid [1-(2,3,5,6-tetramethylbenzyl)indol-4-yl]methylidene hydrazide, 3-chloro-4-hydroxybenzoic acid (4-methoxynaphthalen-1-yl)methylidene hydrazide, or 3-chloro-4-hydroxybenzoic acid [1-(5-chlorothiophen-2-ylmethyl)-1H-indol-5-yl]methylidene hydrazide.
<9> In aspect <6>, the GIP secretion or increase-suppressing agent is preferably 3-bromo-5-methyl-2-phenylpyrazolo[1,5-a]pyrimidin-7-ol, alginic acid, phosphatidylethanolamine, polyglutamic acid, quillaja, lysophosphatidylinositol, cellulose nanofiber, β-chitin nanofiber, diacylglycerol, hydroxypropylated starch, monoacylglycerol, a very long chain fatty acid having 20 or more carbon atoms, triacylglycerol containing 1 mass% or more of docosahexaenoic acid and 1 mass% or more of eicosapentaenoic acid as constituent fatty acids, long chain unsaturated fatty acid ethanolamide, a rice bran extract, a catechin, triacylglycerol containing 10 mass% or more of α-linolenic acid as a constituent fatty acid, or acylglycerol with a C14 to C18 saturated fatty acid bound at the 2-position of the glycerol skeleton.
<10> In aspect <7>, the anti-active GIP antibody is preferably an anti-active GIP antibody that binds to active GIP and does not substantially bind to inactive GIP, wherein the antibody at least recognizes one or more amino acids selected from the 8th to 10th amino acids of the amino acid sequence represented by SEQ ID NO: 5, and includes a region consisting of the amino acid sequence represented by the following formula (1) or a conservative sequence modification thereof in an H-chain:

### EMNPSDGRTHFNE (1).

<11> In aspect <10>, the anti-active GIP antibody is preferably an antibody including a region consisting of the amino acid sequence represented by SEQ ID NO: 2 or a conservative sequence modification thereof as an H-chain variable region.
<12> In aspect <11>, in the anti-active GIP antibody, the conservatively modified amino acid sequence preferably has an identity of 90% or more with the amino acid sequence represented by SEQ ID NO: 2.
<13> In aspect <10>, the anti-active GIP antibody is preferably an antibody including a region consisting of the amino acid sequence represented by SEQ ID NO: 2 or a conservative sequence modification thereof as an H-chain variable region and including a region consisting of the amino acid sequence represented by SEQ ID NO: 4 or a conservative sequence modification as an L-chain variable region.
<14> In aspect <13>, in the anti-active GIP antibody, the amino acid sequence obtained by conservative sequence modification of the amino acid sequence represented by SEQ ID NO: 2 has an identity of 90% or more with the amino acid sequence represented by SEQ ID NO: 2, and the amino acid sequence obtained by conservative sequence modification of the amino acid sequence represented by SEQ ID NO: 4 has an identity of 90% or more with the amino acid sequence represented by SEQ ID NO: 4.

### Examples

### Production Example 1: Preparation of anti-active GIP antibody

### (1) Synthesis of peptide for immunization

Polyethylene glycol was added to N-terminus 15 amino acids of active GIP (GIP(1-15)) (PEGylation (polyethylene glycolation)), and keyhole limpet hemocyanin (KLH) was then chemically bonded thereto to produce KLH-linked PEGylated GIP (1-15) as an immunogen. PEGylated N-terminus 15 amino acids of active GIP (GIP(1-15)) was used as an antigen (1) for measurement, and PEGylated N-terminus 13 amino acids of inactive GIP (GIP(3-15)) was used as an antigen (2) for measurement.

### (2) Immunization

BALB/c mice (Oriental Yeast Co., Ltd.) were immunized subcutaneously in the back. In the first immunization, an emulsion prepared by mixing the antigen prepared as above and Freund's complete adjuvant was administered. Booster immunization was performed with an emulsion prepared by mixing the antigen and Freund's incomplete adjuvant every two weeks from the first immunization. The amount of the antigen used in one immunization was in a range of 0.1 to 0.2 mg. Seven weeks after the first immunization, the antibody titer of the serum collected from each mouse was measured to confirm an increase in the antibody titer.

### (3) Cell fusion

The spleen was excised from the mouse with an increased antibody titer to obtain spleen cells. The obtained spleen cells were fused with mouse myeloma cell line P3U1 by a PEG method. Subsequently, the fused cells were seeded in 20 96-well plates (1×10⁵ cells/well).

### (4) Screening

The reaction between the hybridoma culture supernatant and the antigen (1) and (2) for measurement was evaluated by ELISA using immobilized antigen (1) and (2), and hybridomas that are positive for the antigen (1) and negative for the antigen (2) were selected as anti-active GIP monoclonal antibody-producing hybridomas.

### (5) Cloning

Antibody-producing hybridoma was cloned by culturing the hybridomas obtained above through a limiting dilution method to obtain single colonies, and single colony-forming wells were subjected to ELISA again to establish 9B9H5-B9 line, which produces an antibody that is positive for the antigen (1) and negative for the antigen (2) (WO 2016/104439).

To preserve the resulting antibody-producing hybridomas, the hybridomas were cultured and collected in the logarithmic growth phase and were then prepared to a cell concentration of 1×10⁶ cells/mL with a cryopreservation liquid-containing FBS (fetal bovine serum). The hybridomas were then dispensed into cryogenic tubes at 1×10⁶ cells/tube and were preserved at -80°C in a Bicell.

### (6) Antibody production

The resulting antibody-producing hybridomas in the cryogenic vial were initiated in a hybridoma-SFM (Serum-Free Medium). After amplifying and culturing the hybridomas, culturing was performed in two roller bottles (500 mL × 2, 1 L), and the culture supernatant was collected. The collected culture supernatant was purified to a monoclonal antibody by affinity chromatography using Protein A.

### Test Example 1: Reactivity with active GIP by ELISA

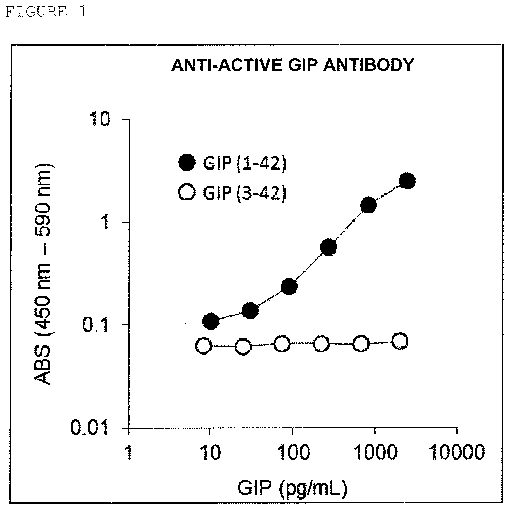

The reactivity between the monoclonal antibody prepared in Production Example 1 and active GIP was confirmed by ELISA. The amino group of the anti-active GIP monoclonal antibody was biotinylated with NH₂ group biotinylation kit (manufactured by Dojindo Laboratories). ELISA was performed using the produced biotinylated anti-active GIP monoclonal antibody at 1 µg/mL instead of a detection antibody, GIP detection antibody (biotinylated anti-total GIP monoclonal antibody), included in Human (total) GIP ELISA kit (manufactured by EMD Millipore Corporation). A 4-fold dilution series of GIP(1-42) or GIP(3-42) was prepared in 6 steps (8.2 to 2000 pg/mL) with a 2000 pg/mL solution as the highest concentration. By using an anti-total GIP monoclonal antibody (included in Human GIP (total) ELISA kit manufactured by EMD Millipore Corporation) as a capture antibody, the biotinylated anti-active GIP monoclonal antibody as a detection antibody, and a peroxidase-streptavidin conjugate for detection, sandwich ELISA was conducted to prepare a calibration curve with GIP concentration on the X-axis and 450 nm - 590 nm absorbance on the Y-axis (Figure 1).

As shown in Figure 1, the absorbance was not increased in GIP(3-42) even in a high-concentration range, and the absorbance was increased only in GIP(1-42) in a concentration-dependent manner. Accordingly, it was verified that the monoclonal antibody prepared in Production Example 1 is an antibody which does not show cross-reactivity with GIP(3-42) and be capable of specifically detecting GIP(1-42).

### Example 1: Acute decrease in body temperature by GIP

### (1) Animal and breeding method

Six-week-old C57BLKS/J misty male mice (m/m mice, Oriental Yeast Co., Ltd.) were transferred (room temperature: 23°C, humidity: 55±10%, light period: 7:00 to 19:00) and were fed with food and water ad libitum. The food was CE-2 (CLEA Japan, Inc.), and the mice were acclimated for 2 weeks under the above-mentioned environment and were then used for testing.

### (2) Preparation of GIP solution

Mouse-derived GIP (manufactured by AnaSpec, Inc.) was dissolved in physiological saline at a concentration of 500 nM to give a GIP solution.

### (3) Administration amount and administration method

Physiological saline (control group) or the GIP solution (5 nmol/kg body weight) (GIP administration group) was intraperitoneally administered to mice (8-week-old) in the light period (9:00 to 10:00 a.m.). Before the administration and 0.5, 2, 4.5, and 8 hours after the administration, a probe was inserted into the rectum of each mouse to measure the body temperature (rectal temperature).

### (4) Body temperature (rectal temperature) measurement

The rectal temperature was measured with a digital rectal thermometer (NS-TC10, manufactured by NeuroScience, Inc.). According to Jikken Dobutsu Handobukku (Handbook of Experimental Animals) (Yokendo CO. Ltd., published in 1983), each mouse was retained under no anesthesia, and the tip of a probe (RET-3 (19 × 0.7 mm shaft diameter), manufactured by Physitemp instruments, LLC) was then inserted into the rectum of the mouse by 0.5 to 1 cm to measure for 15 to 30 seconds.

### (5) Statistical analysis

The analysis results were shown as the average value (Ave.) ± standard error (SE). The statistical analysis was performed using 2-way ANOVA followed by Bonferroni's post hoc test, and the difference was judged to be statistically significant when the P value was 0.05 or less.

### (6) Results

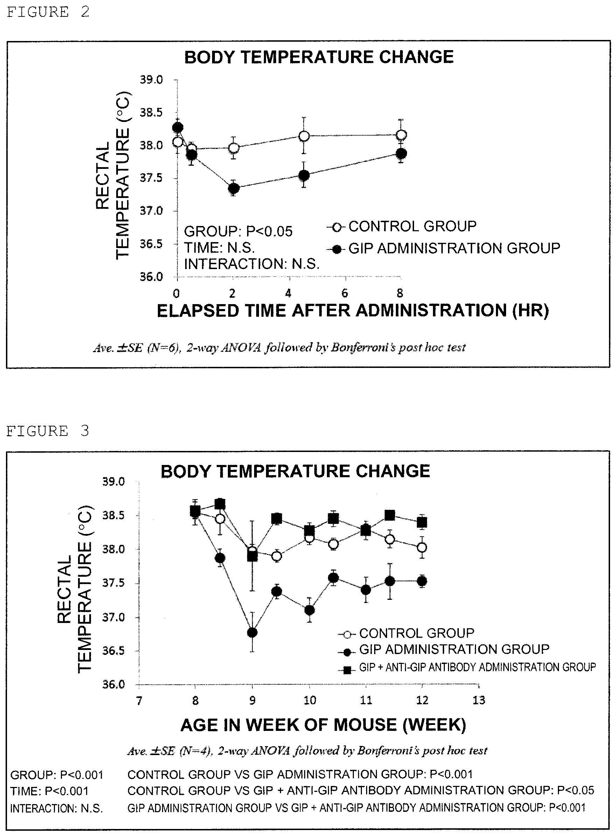

An acute decrease in the body temperature (a decrease of 0.83°C after 2 hours, a decrease of 0.81°C after 4.5 hours) was observed in the GIP administration group, compared to the control group (Figure 2).

### Example 2: Chronic decrease in body temperature by GIP and suppression of decrease in body temperature by anti-GIP antibody

### (1) Animal and breeding method

Six-week-old leptin receptor deficient C57BLKS/J male mice (db/db mice, Oriental Yeast Co., Ltd.) were transferred (room temperature: 23°C, humidity: 55±10%, light period: 7:00 to 19:00) and were fed with food and water ad libitum. The food was CE-2 (CLEA Japan, Inc.), and the mice were acclimated for 2 weeks under the above-mentioned environment and were then used for testing.

### (2) Preparation of GIP solution and GIP-binding anti-GIP antibody solution by antigen antibody reaction

Mouse-derived GIP (manufactured by AnaSpec, Inc.) was dissolved in physiological saline at a concentration of 500 nM to give a GIP solution. Mouse-derived GIP (manufactured by AnaSpec, Inc.) and the anti-active GIP antibody produced in Production Example 1 were dissolved in physiological saline at concentrations of 500 nM and 0.1 mg/mL, respectively, and the resulting solution was incubated for 1 to 2 hours at room temperature to give a GIP-binding anti-GIP antibody solution.

### (3) Administration amount and administration method

Physiological saline (control group), the GIP solution (5 nmol/kg body weight) (GIP administration group), or the GIP-binding anti-GIP antibody solution (GIP: 5 nmol/kg body weight, anti-GIP antibody: 1 mg/kg body weight) (GIP + anti-GIP antibody administration group) was intraperitoneally administered to mice (8-week-old) every morning (9:00 to 10:00 a.m.). The administration period was 28 days, and a probe was inserted into the rectum of each mouse 1 to 2 hours after the administration chronologically (at 3 to 4 days intervals) to measure the body temperature (rectal temperature).

### (4) Body temperature (rectal temperature) measurement

The rectal temperature was measured with a digital rectal thermometer (NS-TC10, manufactured by NeuroScience, Inc.). According to Jikken Dobutsu Handobukku (Handbook of Experimental Animals) (Yokendo CO. Ltd., published in 1983), each mouse was retained under no anesthesia, and the tip of a probe (RET-3 (19 × 0.7 mm shaft diameter), manufactured by Physitemp instruments, LLC) was then inserted into the rectum of the mouse by 0.5 to 1 cm to measure for 15 to 30 seconds.

### (5) Statistical analysis

The analysis results were shown as the average value (Ave.) ± standard error (SE). The statistical analysis was performed using 2-way ANOVA followed by Bonferroni's post hoc test, and the difference was judged to be statistically significant when the P value was 0.05 or less.

### (6) Results

A chronic decrease in the body temperature was observed in the GIP administration group, compared to the control group, and suppression of a decrease in the body temperature was observed in the GIP + anti-GIP antibody administration group (Figure 3).

### Example 3: Age-related change of blood GIP level

### (1) Animal and breeding method

Four-week-old C57BL/6J male mice (CLEA Japan, Inc.) were transferred (room temperature: 23°C, humidity: 55±10%, light period: 7:00 to 19:00) and were fed with food and water ad libitum. The mice were acclimated using CE-2 (CLEA Japan, Inc.) as food for 1 week and were then fed with normal diet (D12450K, Research Diets, Inc.) or high fat diet (D12451, Research Diets, Inc.) for 95 weeks.

### (2) Blood collection

Whole blood of each week old mouse (5-, 10-, 15-, 20-, 30-, 40-, 50-, 65-, 80-, and 100-week-old mice) was collected from the abdominal vena cava under isoflurane anesthesia.

### (3) Measurement of blood GIP level

The collected blood was centrifuged to prepare each plasma fraction, and the blood GIP concentration was then measured according to a usual method with a GIP ELISA kit (manufactured by EMD Millipore Corporation) as the total GIP.

### (4) Statistical analysis

The analysis results were shown as the average value (Ave.) ± standard error (SE). The statistical analysis was performed using 2-way ANOVA followed by Bonferroni's post hoc test, and the difference was judged to be statistically significant when the P value was 0.05 or less.

### (5) Results

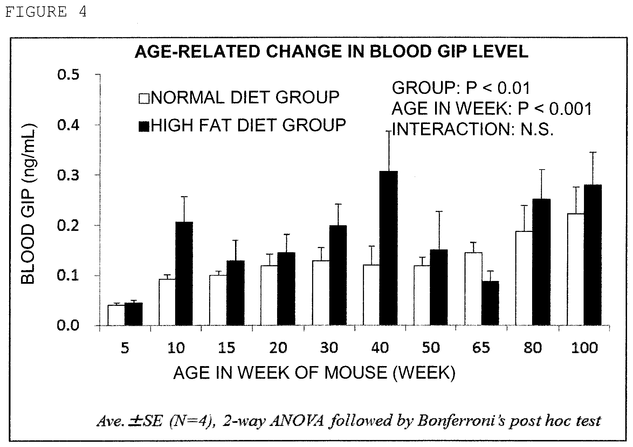

An increase in the blood GIP concentration with aging was observed. In particular, a significant increase in the blood GIP level was observed in the high fat diet group compared to the normal diet group (Figure 4). Since it is known that the total GIP and the amount of active GIP change in conjunction (WO 2012-121302), it is considered that the amount of active GIP is also increased.

### Example 4: Age-related change in deep body temperature (1) Animal and breeding method

Four-week-old C57BL/6J male mice (CLEA Japan, Inc.) were transferred (room temperature: 23°C, humidity: 55±10%, light period: 7:00 to 19:00) and were fed with food and water ad libitum. The mice were acclimated using CE-2 (CLEA Japan, Inc.) as food for 1 week and were then fed with normal diet (D12450K, Research Diets, Inc.) or high fat diet (D12451, Research Diets, Inc.) for 95 weeks. A probe was inserted into the rectum of each mouse every week after the mouse became 5-week-old to measure the body temperature (rectal temperature). The administration period was 8 weeks, and the probe was inserted into the rectum of each mouse chronologically (at 2 to 4 weeks intervals) to measure the body temperature (rectal temperature).

### (2) Body temperature (rectal temperature) measurement

A probe (RET-3 (19 × 0.7 mm shaft diameter), manufactured by Physitemp instruments, LLC) was inserted into the rectum of each mouse every week after the mouse became 5-week-old to measure the body temperature (rectal temperature). The rectal temperature was measured with a digital rectal thermometer (NS-TC10, manufactured by NeuroScience, Inc.). According to Jikken Dobutsu Handobukku (Handbook of Experimental Animals) (Yokendo CO. Ltd., published in 1983), the mouse was retained under no anesthesia, and the tip of the probe was then inserted into the rectum of the mouse by 0.5 to 1 cm to measure for 15 to 30 seconds.

### (3) Statistical analysis

The analysis results were shown as the average value (Ave.) ± standard error (SE). The statistical analysis was performed using 2-way ANOVA followed by Bonferroni's post hoc test, and the difference was judged to be statistically significant when the P value was 0.05 or less.

### (4) Results

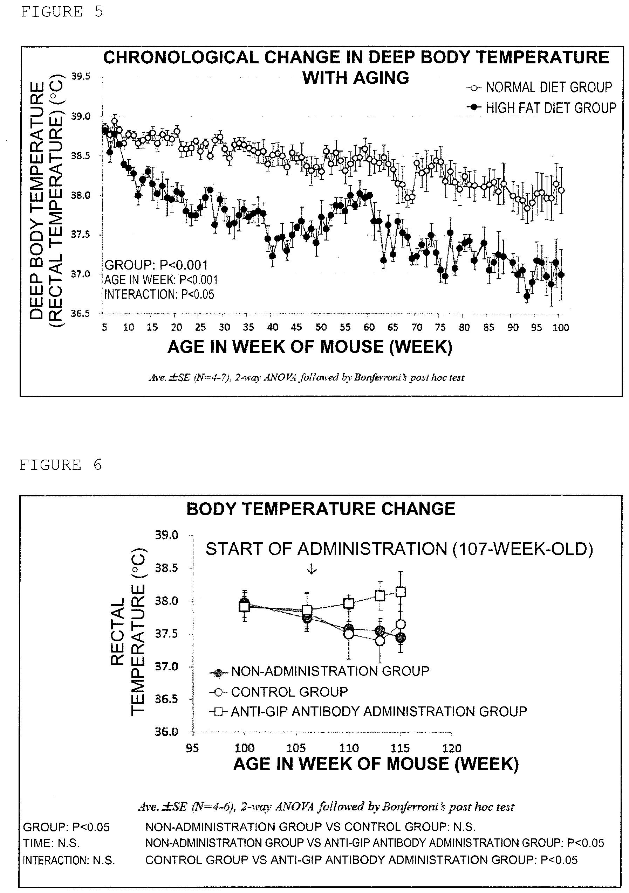

A decrease in the body temperature with aging was observed. In particular, a rapid decrease in the body temperature was observed in the high fat diet intake group compared to the normal diet group (Figure 5).

### Example 5: Suppression of decrease in body temperature of anti-GIP antibody in aged mouse

### (1) Animal and breeding method

Four-week-old C57BL/6J male mice (CLEA Japan, Inc.) were transferred (room temperature: 23°C, humidity: 55±10%, light period: 7:00 to 19:00) and were fed with food (D12450K, Research Diets, Inc.) and water ad libitum for 111 weeks.

### (2) Preparation of anti-GIP antibody solution

The anti-active GIP antibody produced in Production Example 1 was dissolved in physiological saline at a concentration of 0.05 mg/mL to give an anti-GIP antibody solution.

### (3) Administration amount and administration method

Physiological saline (control group) or the anti-GIP antibody solution (0.5 mg/kg body weight) (anti-GIP antibody administration group) was intraperitoneally administered to C57BL/6J mice (107-week-old) once a week (9:00 to 10:00 a.m.). The administration period was 8 weeks, and the probe was inserted into the rectum of each mouse chronologically (at 2 to 4 weeks intervals) to measure the body temperature (rectal temperature).

### (4) Body temperature (rectal temperature) measurement

The rectal temperature was measured with a digital rectal thermometer (NS-TC10, manufactured by NeuroScience, Inc.). According to Jikken Dobutsu Handobukku (Handbook of Experimental Animals) (Yokendo CO. Ltd., published in 1983), each mouse was retained under no anesthesia, and the tip of a probe (RET-3 (19 × 0.7 mm shaft diameter), manufactured by Physitemp instruments, LLC) was then inserted into the rectum of the mouse by 0.5 to 1 cm to measure for 15 to 30 seconds.

### (5) Statistical analysis

The analysis results were shown as the average value (Ave.) ± standard error (SE). The statistical analysis was performed using 2-way ANOVA followed by Bonferroni's post hoc test, and the difference was judged to be statistically significant when the P value was 0.05 or less.

### (6) Results

In the anti-GIP antibody administration group, the body temperature was significantly high compared to those in the control group and the non-administration group, and suppression of a decrease in the body temperature with aging was observed (Figure 6).

### Example 6: Suppression of decease in body temperature by GIP receptor antagonist and GIP secretion or increase-suppressing agent in mouse continuously taking in high fat diet

### (1) Animal and breeding method

Seven-week-old C57BL/6J male mice (CLEA Japan, Inc.) were transferred (room temperature: 23°C, humidity: 55±10%, light period: 7:00 to 19:00) and were fed with food and water ad libitum. The mice were acclimated using CE-2 (CLEA Japan, Inc.) as food for 1 week and were then grouped such that the body weights of each group were equivalent to each other and were fed with a normal diet containing 5% lipid (normal diet group), high fat diet containing 30% lipid (triacylglycerol) (high fat diet group), high fat diet containing 0.4% 4-hydroxybenzoic acid (2-bromobenzylidene) hydrazide (4H2BH, produced by the method described in Ling, et al., J. Med. Chem. 44: 3141-9, 2001) (4H2BH addition group), or high fat diet containing diacylglycerol (JP-A-2006-342084) instead of 30% triacylglycerol (DAG substitution group) for 26 weeks (34-week-old). The food compositions are shown in Table 1. During the breeding period, the body weight was measured once a week, and the food intake amount was measured twice a week. During the breeding period, the intake of the food and water was free. A probe was inserted into the rectum of each mouse at 34-week-old to measure the body temperature (rectal temperature).

**[Table 1]**

| Food composition in food | | | | |
|---|---|---|---|---|
| | Normal diet | High fat diet | | |
| | | TAG | | DAG |
| | | Non-addition (control) | 4H2BH | Non-addition |
| Triacylglycerol (TAG) | 5 | 30 | 30 | 0 |
| Pregelatinized potato starch | 66.5 | 28.5 | 28.1 | 28.5 |
| Sucrose | 0 | 13 | 13 | 13 |
| Casein | 20 | 20 | 20 | 20 |
| Cellulose | 4 | 4 | 4 | 4 |
| Mineral mixture | 3.5 | 3.5 | 3.5 | 3.5 |
| Vitamin mixture | 1 | 1 | 1 | 1 |
| 4H2BH | 0 | 0 | 0.4 | 0 |
| Diacylglycerol (DAG) | 0 | 0 | 0 | 30 |

| | | | | |
|---|---|---|---|---|
| The contents in experimental diet are expressed in percentage (w/w). 4H2BH: 4-Hydroxybenzoic acid (2-bromobenzylidene) hydrazide | | | | |

### (2) Body temperature (rectal temperature) measurement

The rectal temperature was measured with a digital rectal thermometer (NS-TC10, manufactured by NeuroScience, Inc.). According to Jikken Dobutsu Handobukku (Handbook of Experimental Animals) (Yokendo CO. Ltd., published in 1983), each mouse was retained under no anesthesia, and the tip of a probe (RET-3 (19 × 0.7 mm shaft diameter), manufactured by Physitemp instruments, LLC) was then inserted into the rectum of the mouse by 0.5 to 1 cm to measure for 15 to 30 seconds.

### (3) Statistical analysis

The analysis results were shown as the average value (Ave.) ± standard error (SE). The statistical analysis was performed using 1-way ANOVA followed by Dunnett's post hoc test, and the difference was judged to be statistically significant when the P value was 0.05 or less.

### (4) Results

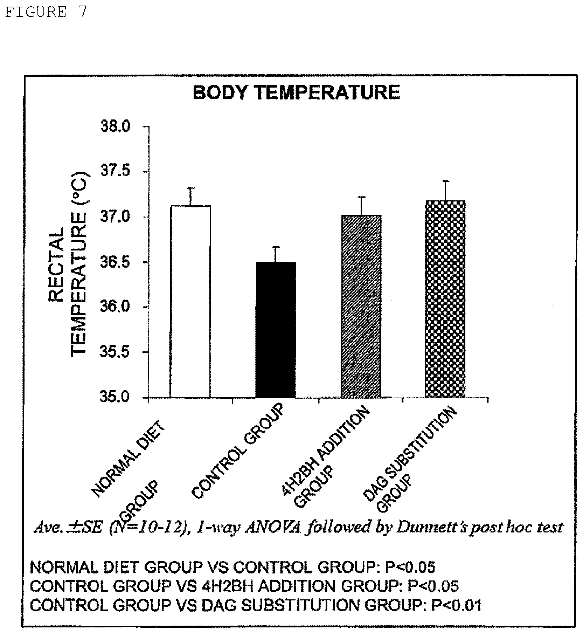

A significant decrease in the body temperature was observed in the group of continuously taking in high fat diet (control group) compared to the normal diet group, and in the groups of taking in a GIP receptor antagonist (4H2BH addition group) or high fat diet containing diacylglycerol substituted for triacylglycerol (DAG substitution group), the decrease in body temperature due to high fat diet intake was suppressed (Figure 7).

## Claims

1. A hypothermia preventing or ameliorating agent comprising a GIP function inhibitor as an active ingredient.

2. The hypothermia preventing or ameliorating agent according to claim 1, wherein the GIP function inhibitor is an anti-GIP antibody, a GIP receptor antagonist, or a GIP secretion or increase-suppressing agent.

3. The hypothermia preventing or ameliorating agent according to claim 2, wherein the anti-GIP antibody is an anti-active GIP antibody.

4. The hypothermia preventing or ameliorating agent according to claim 2, wherein the GIP receptor antagonist is 4-hydroxybenzoic acid (2-bromobenzylidene) hydrazide, 3-cyano-4-hydroxybenzoic acid [1-(2,3,5,6-tetramethylbenzyl)-indol-4-yl]methylidene hydrazide, 3-chloro-4-hydroxybenzoic acid (4-methoxynaphthalen-1-yl)methylidene hydrazide, or 3-chloro-4-hydroxybenzoic acid [1-(5-chlorothiophen-2-ylmethyl)-lH-indol-5-yl]methylidene hydrazide.

5. The hypothermia preventing or ameliorating agent according to claim 2, wherein the GIP secretion or increase-suppressing agent is 3-bromo-5-methyl-2-phenylpyrazolo[1,5-a]pyrimidin-7-ol, alginic acid, phosphatidylethanolamine, polyglutamic acid, quillaja, lysophosphatidylinositol, cellulose nanofiber, β-chitin nanofiber, diacylglycerol, hydroxypropylated starch, monoacylglycerol, a very long chain fatty acid having 20 or more carbon atoms, triacylglycerol containing 1 mass% or more docosahexaenoic acid and 1 mass% or more of eicosapentaenoic acid as constituent fact acids, long chain unsaturated fatty acid ethanolamide, a rice bran extract, a catechin, triacylglycerol containing 10 mass% or more of α-linolenic acid as a constituent fatty acid, or acylglycerol with a C14 to C18 saturated fatty acid bound at the 2-position of the glycerol skeleton.

6. Use of a GIP function inhibitor for producing a hypothermia preventing or ameliorating agent.

7. The use according to claim 6, wherein the GIP function inhibitor is an anti-GIP antibody, a GIP receptor antagonist, or a GIP secretion or increase-suppressing agent.

8. The use according to claim 7, wherein the anti-GIP antibody is an anti-active GIP antibody.

9. The use according to claim 7, wherein the GIP receptor antagonist is 4-hydroxybenzoic acid (2-bromobenzylidene) hydrazide, 3-cyano-4-hydroxybenzoic acid [1-(2,3,5,6-tetramethylbenzyl)-indol-4-yl]methylidene hydrazide, 3-chloro-4-hydroxybenzoic acid (4-methoxynaphthalen-1-yl)methylidene hydrazide, or 3-chloro-4-hydroxybenzoic acid [1-(5-chlorothiophen-2-ylmethyl)-1H-indol-5-yl]methylidene hydrazide.

10. The use according to claim 7, wherein the GIP secretion or increase-suppressing agent is 3-bromo-5-methyl-2-phenylpyrazolo[1,5-a]pyrimidin-7-ol, alginic acid, phosphatidylethanolamine, polyglutamic acid, quillaja, lysophosphatidylinositol, cellulose nanofiber, β-chitin nanofiber, diacylglycerol, hydroxypropylated starch, monoacylglycerol, a very long chain fatty acid having 20 or more carbon atoms, triacylglycerol containing 1 mass% or more of docosahexaenoic acid and 1 mass% or more of eicosapentaenoic acid as constituent fatty acids, long chain unsaturated fatty acid ethanolamide, a rice bran extract, a catechin, triacylglycerol containing 10 mass% or more of α-linolenic acid as constituent fatty acid, or acylglycerol with a C14 to C18 saturated fatty acid bound at the 2-position of the glycerol skeleton.

11. A GIP function inhibitor for use in prevention or amelioration of hypothermia.

12. The GIP function inhibitor according to claim 11, wherein the GIP function inhibitor is an anti-GIP antibody, a GIP receptor antagonist, or a GIP secretion or increase-suppressing agent.

13. The GIP function inhibitor according to claim 12, wherein the anti-GIP antibody is an anti-active GIP antibody.

14. The GIP function inhibitor according to claim 12, wherein the GIP receptor antagonist is 4-hydroxybenzoic acid (2-bromobenzylidene) hydrazide, 3-cyano-4-hydroxybenzoic acid [1-(2,3,5,6-tetramethylbenzyl)-indol-4-yl]methylidene hydrazide, 3-chloro-4-hydroxybenzoic acid (4-methoxynaphthalen-1-yl)methylidene hydrazide, or 3-chloro-4-hydroxybenzoic acid [1-(5-chlorothiophen-2-ylmethyl)-1H-indol-5-yl]methylidene hydrazide.

15. The GIP function inhibitor according to claim 12, wherein the GIP secretion or increase-suppressing agent is 3-bromo-5-methyl-2-phenylpyrazolo[1,5-a]pyrimidin-7-ol, alginic acid, phosphatidylethanolamine, polyglutamic acid, quillaja, lysophosphatidylinositol, cellulose nanofiber, β-chitin nanofiber, diacylglycerol, hydroxypropylated starch, monoacylglycerol, a very long chain fatty acid having 20 or more carbon atoms, triacylglycerol containing 1 mass% or more of docosahexaenoic acid and 1 mass% or more of eicosapentaenoic acid as constituent fatty acids, long chain unsaturated fatty acid ethanolamide, a rice bran extract, a catechin, triacylglycerol containing 10 mass% or more of α-linolenic acid as a constituent fatty acid, or acylglycerol with a C14 to C18 saturated fatty acid bound at the 2-position of the glycerol skeleton.

16. Use of a GIP function inhibitor for preventing or ameliorating hypothermia.

17. The use according to claim 16, wherein the GIP function inhibitor is an anti-GIP antibody, a GIP receptor antagonist, or a GIP secretion or increase-suppressing agent.

18. The use according to claim 17, wherein the anti-GIP antibody is an anti-active GIP antibody.

19. The use according to claim 17, wherein the GIP receptor antagonist is 4-hydroxybenzoic acid (2-bromobenzylidene) hydrazide, 3-cyano-4-hydroxybenzoic acid [1-(2,3,5,6-tetramethylbenzyl)-indol-4-yl]methylidene hydrazide, 3-chloro-4-hydroxybenzoic acid (4-methoxynaphthalen-1-yl)methylidene hydrazide, or 3-chloro-4-hydroxybenzoic acid [1-(5-chlorothiophen-2-ylmethyl)-1H-indol-5-yl]methylidene hydrazide.

20. The use according to claim 17, wherein the GIP secretion or increase-suppressing agent is 3-bromo-5-methyl-2-phenylpyrazolo[1,5-a]pyrimidin-7-ol, alginic acid, phosphatidylethanolamine, polyglutamic acid, quillaja, lysophosphatidylinositol, cellulose nanofiber, β-chitin nanofiber, diacylglycerol, hydroxypropylated starch, monoacylglycerol, a very long chain fatty acid having 20 or more carbon atoms, triacylglycerol containing 1 mass% or more of docosahexaenoic acid and 1 mass% or more of eicosapentaenoic acid as constituent fatty acids, long chain unsaturated fatty acid ethanolamide, a rice bran extract, a catechin, triacylglycerol containing 10 mass% or more of α-linolenic acid as a constituent fatty acid, or acylglycerol with a C14 to C18 saturated fatty acid bound at the 2-position of the glycerol skeleton.

21. A method for preventing or ameliorating hypothermia, comprising administering a GIP function inhibitor to a subject in need thereof.

22. The method according to claim 21, wherein the GIP function inhibitor is an anti-GIP antibody, a GIP receptor antagonist, or a GIP secretion or increase-suppressing agent.

23. The method according to claim 22, wherein the anti-GIP antibody is an anti-active GIP antibody.

24. The method according to claim 22, wherein the GIP receptor antagonist is 4-hydroxybenzoic acid (2-bromobenzylidene) hydrazide, 3-cyano-4-hydroxybenzoic acid [1-(2,3,5,6-tetramethylbenzyl)-indol-4-yl]methylidene hydrazide, 3-chloro-4-hydroxybenzoic acid (4-methoxynaphthalen-1-yl)methylidene hydrazide, or 3-chloro-4-hydroxybenzoic acid [1-(5-chlorothiophen-2-ylmethyl)-1H-indol-5-yl]methylidene hydrazide.

25. The method according to claim 22, wherein the GIP secretion or increase-suppressing agent is 3-bromo-5-methyl-2-phenylpyrazolo[1,5-a]pyrimidin-7-ol, alginic acid, phosphatidylethanolamine, polyglutamic acid, quillaja, lysophosphatidylinositol, cellulose nanofiber, β-chitin nanofiber, diacylglycerol, hydroxypropylated starch, monoacylglycerol, a very long chain fatty acid having 20 or more carbon atoms, triacylglycerol containing 1 mass% or more of docosahexaenoic acid and 1 mass% or more of eicosapentaenoic acid as constituent fatty acids, long chain unsaturated fatty acid ethanolamide, a rice bran extract, a catechin, triacylglycerol containing 10 mass% or more of α-linolenic acid as a constituent fatty acid, or acylglycerol with a C14 to C18 saturated fatty acid bound at the 2-position of the glycerol skeleton.


SEQUENCE LISTING

<110>  KAO CORPORATION

<120>  AGENT FOR IMPROVING HYPOTHERMIA

<130>  KS1541

<150>  JP 2016-251776
<151>  2016-12-26

<160>  5

<170>  PatentIn version 3.5

<210>  1
<211>  339
<212>  DNA
<213>  Mus musculus

<220>
<221>  CDS
<222>  (1)..(339)

<400>  1
cag gtc caa ctg cag cag cct ggg gct gaa ctg gtg aag cct ggg gcc      48
Gln Val Gln Leu Gln Gln Pro Gly Ala Glu Leu Val Lys Pro Gly Ala
1               5                   10                  15

tca gtg aag ctg tcc tgc aag gct tct ggc tac acc ttc acc agc ttc      96
Ser Val Lys Leu Ser Cys Lys Ala Ser Gly Tyr Thr Phe Thr Ser Phe
            20                  25                  30

tgg atg cac tgg gtg att cag agg cct gga caa ggc ctt gag tgg att      144
Trp Met His Trp Val Ile Gln Arg Pro Gly Gln Gly Leu Glu Trp Ile
        35                  40                  45

gga gag atg aat cct agc gac ggt cgt act cac ttc aat gaa aag ttc      192
Gly Glu Met Asn Pro Ser Asp Gly Arg Thr His Phe Asn Glu Lys Phe
    50                  55                  60

aag acc aag gcc aca ctg act ata gac aca tcc tcc aac aca gcc tac      240
Lys Thr Lys Ala Thr Leu Thr Ile Asp Thr Ser Ser Asn Thr Ala Tyr
65                  70                  75                  80

atg gaa ctc aac agc ctg aca tct gag gac tct gcg gtc tat tac tgt      288
Met Glu Leu Asn Ser Leu Thr Ser Glu Asp Ser Ala Val Tyr Tyr Cys
                85                  90                  95

gca aga agg atg gag gac tgg ggc caa ggg act ctg gtc act gtt tct      336
Ala Arg Arg Met Glu Asp Trp Gly Gln Gly Thr Leu Val Thr Val Ser
            100                 105                 110

gca                                                                  339
Ala

<210>  2
<211>  113
<212>  PRT
<213>  Mus musculus

<400>  2

Gln Val Gln Leu Gln Gln Pro Gly Ala Glu Leu Val Lys Pro Gly Ala
1               5                   10                  15

Ser Val Lys Leu Ser Cys Lys Ala Ser Gly Tyr Thr Phe Thr Ser Phe
            20                  25                  30

Trp Met His Trp Val Ile Gln Arg Pro Gly Gln Gly Leu Glu Trp Ile
        35                  40                  45

Gly Glu Met Asn Pro Ser Asp Gly Arg Thr His Phe Asn Glu Lys Phe
    50                  55                  60

Lys Thr Lys Ala Thr Leu Thr Ile Asp Thr Ser Ser Asn Thr Ala Tyr
65                  70                  75                  80

Met Glu Leu Asn Ser Leu Thr Ser Glu Asp Ser Ala Val Tyr Tyr Cys
                85                  90                  95

Ala Arg Arg Met Glu Asp Trp Gly Gln Gly Thr Leu Val Thr Val Ser
            100                 105                 110

Ala

<210>  3
<211>  324
<212>  DNA
<213>  Mus musculus

<220>
<221>  CDS
<222>  (1)..(324)

<400>  3
gac atc aag atg acc cag tct cca tct tcc atg tat gca tct cta gga      48
Asp Ile Lys Met Thr Gln Ser Pro Ser Ser Met Tyr Ala Ser Leu Gly
1               5                   10                  15

gag aga gtc act atc act tgc aag gcg agt cag gac att aat agc tat      96
Glu Arg Val Thr Ile Thr Cys Lys Ala Ser Gln Asp Ile Asn Ser Tyr
            20                  25                  30

tta ggc tgg ttc cag cag aaa cca ggg aaa tct cct aag acc ctg ata      144
Leu Gly Trp Phe Gln Gln Lys Pro Gly Lys Ser Pro Lys Thr Leu Ile
        35                  40                  45

tat ggt gca aac aga ttg gta gat ggg gtc cca tca agg ttc agt ggc      192
Tyr Gly Ala Asn Arg Leu Val Asp Gly Val Pro Ser Arg Phe Ser Gly
    50                  55                  60

agt gga tct ggg caa gat tac tct ctc acc atc agc agc ctg gag tat      240

Ser Gly Ser Gly Gln Asp Tyr Ser Leu Thr Ile Ser Ser Leu Glu Tyr
65                  70                  75                  80

gac gat atg gga ata tat tat tgt cta cag tat gat gag ttt ccg ctc       288
Asp Asp Met Gly Ile Tyr Tyr Cys Leu Gln Tyr Asp Glu Phe Pro Leu
                85                  90                  95

acc ttc ggt gct ggg acc aag ctg gag ctg aaa cgg                       324
Thr Phe Gly Ala Gly Thr Lys Leu Glu Leu Lys Arg
            100                 105

<210>  4
<211>  108
<212>  PRT
<213>  Mus musculus

<400>  4

Asp Ile Lys Met Thr Gln Ser Pro Ser Ser Met Tyr Ala Ser Leu Gly
1               5                   10                  15

Glu Arg Val Thr Ile Thr Cys Lys Ala Ser Gln Asp Ile Asn Ser Tyr
            20                  25                  30

Leu Gly Trp Phe Gln Gln Lys Pro Gly Lys Ser Pro Lys Thr Leu Ile
        35                  40                  45

Tyr Gly Ala Asn Arg Leu Val Asp Gly Val Pro Ser Arg Phe Ser Gly
    50                  55                  60

Ser Gly Ser Gly Gln Asp Tyr Ser Leu Thr Ile Ser Ser Leu Glu Tyr
65                  70                  75                  80

Asp Asp Met Gly Ile Tyr Tyr Cys Leu Gln Tyr Asp Glu Phe Pro Leu
                85                  90                  95

Thr Phe Gly Ala Gly Thr Lys Leu Glu Leu Lys Arg
            100                 105

<210>  5
<211>  42
<212>  PRT
<213>  Homo sapiens

<400>  5

Tyr Ala Glu Gly Thr Phe Ile Ser Asp Tyr Ser Ile Ala Met Asp Lys
1               5                   10                  15

Ile His Gln Gln Asp Phe Val Asn Trp Leu Leu Ala Gln Lys Gly Lys
            20                  25                  30

Lys Asn Asp Trp Lys His Asn Ile Thr Gln

35                                                    40